# EUROPÄISCHE PATENTSCHRIFT

(11) **EP 1 195 394 B1**
(45) Veröffentlichungstag und Bekanntmachung des Hinweises auf die Patenterteilung: **11.08.2004**
(21) Anmeldenummer: 01123521.5
(22) Anmeldetag: 29.09.2001
(51) Int. Cl.: C08F 261/04, A61K 9/32

(54) **(Co-)Polymerisate von Hydroxyalkyl(meth)acrylaten, Verfahren zu deren Herstellung sowie deren Verwendung in pharmazeutischen Darreichungsformen**
(Co-)Polymers of hydroxyalkyl(meth)acrylates, process for their preparation and their use in pharmaceutical dosage forms
Polymères et copolymères à base d'hydroxyacrylates ou hydroxymethacrylates, leur préparation et leur utilisation pour les formes posologiques en pharmacie

(30) Priorität: 04.10.2000 DE 10049297
(43) Veröffentlichungstag der Anmeldung: 10.04.2002
(73) Patentinhaber: BASF AKTIENGESELLSCHAFT, 67056 Ludwigshafen (DE)
(72) Erfinder: Angel, Maximilian, Dr., 67105 Schifferstadt (DE); Sanner, Axel, Dr., 67227 Frankenthal (DE); Kolter, Karl, Dr., 67117 Limburgerhof (DE)

(56) Entgegenhaltungen:
- EP-A- 0 821 016
- WO-A-00/44356
- WO-A-92/07553
- GB-A- 1 278 813
- US-A- 4 397 968
- US-A- 4 581 394
- US-A- 5 354 803

## Beschreibung

Die vorliegende Erfindung betrifft wasserlösliche oder wasserdispergierbare Copolymerisate von Hydroxyalkyl(meth)acrylaten, Verfahren zu deren Herstellung sowie deren Verwendung als Überzugs-, Bindemittel und/oder filmbildender Hilfsstoff in pharmazeutischen Darreichungsformen.

Feste pharmazeutische Darreichungsformen wie Tabletten, Kapseln, Pellets, Granulate, Kristalle etc. werden aus sehr unterschiedlichen Gründen gecoated, d.h. mit einem Filmüberzug versehen. So kann beispielsweise ein schlechter Geruch oder Geschmack maskiert sowie die Schluckbarkeit verbessert werden. Die Stabilität des Wirkstoffes kann durch das Coating erhöht werden, indem weniger Wasserdampf und Sauerstoff an das Tabletteninnere gelangt. Die Darreichungsformen sehen besser aus und können durch die Einarbeitung von Farbstoffen besser unterschieden werden. Darüber hinaus läßt sich insbesondere die Freisetzungsgeschwindigkeit des Wirkstoffes durch den Filmüberzug einstellen.

Generell unterscheidet man Instant-Release-Formen und Retard- bzw. Slow-Release-Formen.

Bei Instant-Release-Formen sollen der Zerfall der Tablette und die Freisetzung des Wirkstoffes aus der Darreichungsform nach Möglichkeit nicht durch das Coating beeinflußt werden, deshalb muß sich der Filmüberzug schnell im Magensaft auflösen. Daneben muß er über gute Filmeigenschaften verfügen. Die Zugfestigkeit und die Reißdehnung sollten hoch sein, damit der Filmüberzug mechanischen Einwirkungen standhält, wie sie bei der pharmazeutischen Verarbeitung - insbesondere der Konfektionierung - und auch während des Versandes bzw. der Lagerung auftreten.

Ein häufig eingesetztes Produkt für das Coaten von Instant-Release-Tabletten ist Hydroxypropylmethylcellulose (HPMC). Hydroxypropylmethylcellulose weist in wäßriger Lösung bei zunehmender Konzentration einen steilen Viskositätsanstieg auf. Ein ähnliches Verhalten zeigt auch Hydroxypropylcellulose (HPC).

Da die Filmbildnerlösung beim Coaten von Tabletten fein zerstäubt werden muß und die gebildeten Tröpfchen die Oberfläche der Tabletten gut benetzen und auch gut spreiten müssen, darf die Viskosität eine gewisse Grenze (zwischen 150 und 250 mPas), die abhängig ist von der Art der Sprühdüse und des Gerätes, nicht überschreiten. Deshalb können im Falle von HPMC nur verhältnismäßig niedrige Filmbildnerkonzentrationen eingesetzt werden.

Als Empfehlung für die Konzentration von Pharmacoat® 606 (Fa. Shin-etsu) werden in der Literatur 5 bis 7 Gew.-% angegeben (Pharmaceutical Coating Technology, edited by Graham Cole, Taylor and Francis Ltd. 1995 und Technische Merkblätter der Hersteller). Diese geringe Sprühkonzentration bedingt eine relativ lange Verarbeitungszeit und damit hohe Kosten.

Darüberhinaus zeigt Hydroxypropylmethylcellulose weitere Nachteile u.a. im Benetzungsverhalten, in der Adhesivität auf der Tablettenoberfläche, im Pigmentbindevermögen, in den mechanischen Eigenschaften der Filme, in der Hygroskopizität sowie in der Permeabilität gegenüber Wasserdampf und Sauerstoff, in der Auflösungsgeschwindigkeit und in der Zerfallszeitdifferenz zwischen Filmtablette und Kern.

Die geringe Elastizität der Filme aus Hydroxypropylmethylcellulose führt häufig dazu, daß die Filmtabletten bei feuchter Lagerung infolge der Quellung des Kerns aufreißen. Auch der Einsatz von Weichmachern ergibt keine nennenswerten Verbesserungen dieses Problems. Es kann vielmehr zu klebrigen Filmen und durch Migration zu Veränderungen der Tabletteneigenschaften führen.

Bindemittel werden in pharmazeutischen Darreichungsformen eingesetzt, um die Verarbeitbarkeit und die mechanische Festigkeit zu erhöhen. Sie werden üblicherweise in Tabletten, Granulaten und Pellets eingesetzt und führen zu verbesserter Fließfähigkeit, höherer Bruchfestigkeit und geringerer Friabilität.

Die derzeit verwendeten Bindemittel wie Maltodextrin oder Polyvinylpyrrolidon führen häufig zu nicht zufriedenstellenden Bruchfestigkeiten und Friabilitäten. Andere Bindemittel wie Stärkekleister und Hydroxypropylmethylcellulose (HPMC) lassen sich aufgrund ihrer hohen Viskosität nur niedrigkonzentriert einsetzen.

Weiterhin werden filmbildende Hilfsstoffe in Lösungen und Sprays eingesetzt, die auf der Haut oder Schleimhaut aufgebracht oder auch dem Körper systemisch zugeführt werden. Beispiele hierfür sind Zubereitungen für die Wundbehandlung, Sprayverbände aber auch Zubereitungen zur Applikation auf intakter Haut bzw. Schleimhaut. Dabei wird die Haut durch einen Film geschützt und die Wirkstoffe können in bzw. durch die Haut dringen.

Bei transdermalen therapeutischen Systemen und bei Wundpflastern ist ebenfalls wie bei den oben genannten Darreichungsformen eine hohe Flexibilität erforderlich, die die derzeit zur Verfügung stehenden Produkte nicht aufweisen. Der Einsatz von möglichen Weichmachern zur Erreichung der notwendigen Flexibilität ist aus toxikologischen und pharmakologischen Gründen nicht erwünscht.

In GB 1 278 813 werden Acrylat-Emulsionspolymerisate beschrieben, die sich durch eine hohe Wasserresistenz gegenüber konventionellen Seifendispersionen auszeichnen, was sie für die Verwendung in Instant-Release-Tabletten unbrauchbar macht.

DE 31 11 602 beschreibt durch Polyvinylalkohol stabilisierte Emusionspolymerisate, die zu mindestens 60 Gew.-% aus (Meth-) Acrylat- und/oder Styrol-Einheiten bestehen. Verwendet werden sie als Bindemittel für Dispersionsfarben und Klebstoffe.

In DE-A 196 29 948 werden Dispersionen offenbart, in denen zwingend Styrol enthalten ist, und die als Einsatzstoffe in Baustoffen verwendet werden.

Der vorliegenden Erfindung lag die Aufgabe zugrunde, wasserlösliche oder wasserdispergierbare Polymere als Überzugsmittel, Bindemittel und/oder filmbildende Hilfsstoffe in pharmazeutischen Darreichungsformen, insbesondere für Instant-Release-Formen bereitzustellen, die die oben genannten Nachteile nicht aufweisen.

Die Aufgabe wurde erfindungsgemäß gelöst durch wasserlösliche oder wasserdispergierbare Copolymerisate, die erhältlich sind durch radikalisch initiierte Polymerisation, bevorzugt Emulsionspolymerisation von
a) 80 bis 20 Gew.-% Hydroxy-C₁-C₆-alkyl(meth)acrylat sowie gegebenenfalls einer oder mehrerer Verbindungen der Formel (A) oder (B) 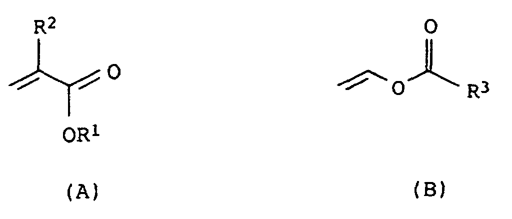 mit R¹ = H, C₁-C₆-Alkyl,
   R² = H, CH₃
   R³ = C₁-C₂₄-Alkyl
   oder Gemischen davon
   in Gegenwart von
b) 20 bis 80 Gew.-% Polyvinylalkohol (PVA) und
c) gegebenenfalls 0 bis 20 Gew.-% weiterer polymerisierbarer Verbindungen (C).

Ein weiterer Gegenstand der Erfindung ist ein Verfahren zur Herstellung der Copolymerisate durch radikalisch initiierte Polymerisation, bevorzugt Emulsionspolymerisation, in einem wäßrigen oder nichtwäßrigen, aber wassermischbaren Lösungsmittel oder in gemischt nichtwäßrigen/wäßrigen Lösungsmitteln. Bevorzugt ist die Herstellung in Wasser als Lösungs- bzw. Dispersionsmittel.

Geeignete nichtwäßrige Lösungsmittel sind beispielsweise Alkohole, wie Methanol, Ethanol, n-Propanol, und Isopropanol sowie Glykole, wie Ethylenglykol und Glycerin.

Als Hydroxy-C₁-C₆-alkyl(meth)acrylat werden bevorzugt Hydroxymethylmethacrylat, Hydroxyethylmethacrylat, Hydroxypropylmethacrylat, Hydroxybutylmethacrylat, Hydroxypentylmethacrylat, insbesondere bevorzugt Hydroxyethylmethacrylat eingesetzt. Als Verbindungen der Formel (A) werden bevorzugt C₁-C₆-Alkyl(meth)acrylate, insbesondere Methylmethacrylat, Ethylacrylat und Methylacrylat oder Gemische davon, eingesetzt. Als Verbindungen der Formel B werden C₃-C₂₄-Vinylester, insbesondere Vinylacetat eingesetzt. Die Verbindungen der Formel A sind bevorzugt genenüber Verbindungen der Formel (B).

Als Verbindungen (C) kommen bevorzugt in Frage: Acryl- und Methacrylsäure. Weitere Verbindungen (C) sind Crotonsäure, Maleinsäuremono(C₁-C₈)-alkylester, Maleinsäure, Fumarsäure, Itaconsäure, (Meth-)Acrylnitril, ethylenisch ungesättigte Dicarbonsäuredi(C₁-C₂₂)-alkylester, vorzugsweise Maleinsäurebutylester, ethylenisch ungesättigte Sulfonsäuren oder Sulfonsäurederivate wie Vinylsulfonsäure oder deren Alkalisalze. Acyclische N-Vinylcarbonsäureamide und N-Vinyllactame wie Vinylpyrrolidon.

Auch mehrfach ethylenisch ungesättigte copolymerisationsfähige Verbindungen, die vernetzend wirken können enthalten sein, vorzugsweise aus der Gruppe Divinylbenzol, Diallylphthalat, Butandioldiacrylat, Butandioldimethacrylat. Weitere geeignete vernetzende Monomere sind z.B. in der DE 197 12 247 A1, Seite 5, genannt. Bevorzugt beträgt der Anteil an Verbindungen (C) aber 0 Gew.-%.

Eine bevorzugte Ausführungsform der Erfindung sind wasserlösliche oder wasserdispergierbare Copolymerisate, die erhältlich sind durch radikalisch initiierte Polymerisation, bevorzugt Emulsionspolymerisation von
a) 80 bis 20 Gew.-% Hydroxy-C₁-C₆-alkyl(meth)acrylat sowie einer oder mehrerer Verbindungen der Formel (A) oder (B) 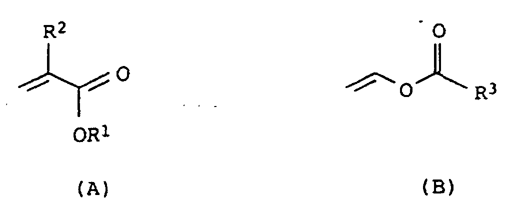 mit R¹ = H, C₁-C₆-Alkyl,
   R² = H, CH₃
   R³ = C₁-C₂₄-Alkyl
   oder Gemischen davon
   in Gegenwart von
b) 20 bis 80 Gew.-% Polyvinylalkohol (PVA) und
c) gegebenenfalls 0 bis 20 Gew.-% weiterer polymerisierbarer Verbindungen (C), wobei der Gew.-%-Anteil an Hydroxy-C₁-C₆alkyl(meth)acrylat wenigstens einmal, bevorzugt zweimal, insbesondere dreimal so groß ist wie der Gew.-%-Anteil an den Verbindungen der Formel (A) oder (B).

Als Polyvinylalkohole (PVA) kommen vorzugsweise teilverseifte, aber auch vollverseifte, (kalt-) wasserlösliche PVA mit Molekulargewichten zwischen etwa 2000 und etwa 250000, insbesondere etwa 10000 bis 100000, wie sie durch Alkoholyse oder Hydrolyse von Polyvinylestern, vorzugsweise von Polyvinylacetaten erhalten werden in Frage. Bevorzugt sind PVA mit einem Verseifungsgrad von 65 bis 99 %, insbesondere bevorzugt von 80 bis 90 %.

Die Polymerisation findet bevorzugt in Gegenwart von 20 bis 80 Gew.-%, bevorzugt von 25 bis 60 Gew.-% insbesondere von 30 bis 55 Gew.-% Polyvinylalkohol statt. Der "Rest" zu 100 Gew.-% entfällt jeweils auf die Verbindungen Hydroxy-C₁-C₆-alkyl(meth)acrylat, A und/oder B bzw. (C).

Werden neben Hydroxy-C₁-C₆-alkyl(meth)acrylaten eine oder mehrere Verbindungen der Formel A oder B eingesetzt, so ist der Gew.-%-Anteil an Hydroxy-C₁-C₆-alkyl(meth)acrylsäureestern wenigstens einmal, bevorzugt wenigstens zweimal, insbesondere bevorzugt wenigstens dreimal so groß wie der Gew.-%-Anteil an den Verbindungen der Formel (A) oder (B).

Zur Herstellung der Polymerisate können die Monomeren der Formel A und/oder B und/oder C in Gegenwart des PVA sowohl mit Hilfe von Radikale bildenden Initiatoren als auch durch Einwirkung energiereicher Strahlung, worunter auch die Einwirkung energiereicher Elektronen verstanden werden soll, polymerisiert werden.

Die Emulsionspolymerisation wird vorzugsweise bei Temperaturen von 60 bis 100°C durchgeführt.

Zur Initiierung der Emulsionspolymerisation werden radikalische Initiatoren eingesetzt. Die verwendeten Mengen an Initiator bzw. Initiatorgemischen bezogen auf eingesetztes Monomer liegen zwischen 0,01 und 10 Gew.-%, vorzugsweise zwischen 0,3 und 5 Gew.-%.

Je nach Art des verwendeten Lösungsmittels eignen sich sowohl organische als auch anorganische Peroxide oder Azostarter wie Azo-bis-isobutyronitril, Azo-bis-(2-amidopropan)dihydrochlorid oder 2,2'-Azo-bis-(2-methyl-butyronitril).

Peroxidische Initiatoren sind beispielsweise Dibenzoylperoxid, Diacetylperoxid, Succinylperoxid, tert.-Butylperpivalat, tert.-Butyl-2-ethylhexanoat, tert.-Butylpermaleinat, Bis-(tert.-Butylperoxi)-cyclohexan, tert.-Butylperoxi-isopropylcarbonat, tert.-Butylperacetat, 2,2-Bis-(tert.-butylperoxi)-butan, Dicumylperoxid, Di-tert.-amylperoxid, Di-tert.-butylperoxid, p-Menthanhydroperoxid, Pinanhydroperoxid, Cumolhydroperoxid, tert.-Butylhydroperoxid, Wasserstoffperoxid sowie Mischungen der genannten Initiatoren. Die genannten Initiatoren können auch in Kombination mit Redoxkomponenten wie Ascorbinsäure verwendet werden.

Besonders geeignet als Initiator sind Alkali- oder Ammoniumpersulfat.

Die radikalische Emulsionspolymerisation findet vorzugsweise in Wasser unter Mitverwendung von Polyvinylalkohol, in Gegenwart von radikalbildenden Polymerisationsinitiatoren, gegebenenfalls Emulgatoren, gegebenenfalls weiteren Schutzkolloiden, gegebenenfalls Molekulargewichtsreglern, gegebenenfalls Puffersystemen und gegebenenfalls nachfolgender pH-Einstellung mittels Basen oder Säuren statt. Die Copolymerisate werden als wäßrige Dispersionen oder wäßrige Lösungen mit einer Viskosität kleiner als 500 mPas, bevorzugt kleiner 250 mPas, besonders bevorzugt kleiner 150 mPas, oder nach Entfernung des Wasseranteils, als wasserdispergierbare oder wasserlösliche Pulver, gewonnen.

Geeignete Schutzkolloide, neben PVA, sind wasserlösliche Cellulosederivate, vorzugsweise aus der Gruppe Hydroxyethylcellulose, Carboxymethylcellulose, Carboxymethylhydroxyethylcellulose, Methylhydroxyethylcellulose, oder wasserlösliche (Co-)Polymere aus N-Vinylamidverbindungen oder N-Vinyllactamverbindungen, vorzugsweise Polyvinylpyrrolidon (PVP), oder wasserlösliche polymere, copolymere oder blockcopolymere Polyalkylenoxide, vorzugsweise des Ethylenoxids und/oder des Propylenoxids.

Als Molekulargewichtsregler eignen sich Schwefelwasserstoffverbindungen wie Alkylmercaptane, z.B. n-Dodecylmercaptan, tert.-Dodecylmercaptan, Thioglykolsäure und deren Ester, Mercaptoalkanole wie Mercaptoethanol. Weitere geeignete Regler sind z.B. in der DE 197 12 247 A1, Seite 4, genannt. Die erforderliche Menge der Molekulargewichtsregler liegt im Bereich von 0 bis 5 Gew.-% bezogen auf die zu polymerisierenden (Co-)Monomerenmenge, insbesondere 0,05 bis 2 Gew.-%, besonders bevorzugt 0,1 bis 1,5 Gew.-%.

Als Emulgatoren verwendet man beispielsweise ionische oder nichtionische Tenside, deren HLB-Wert üblicherweise im Bereich von 3 bis 13 liegt. Zur Definition des HLB-Werts wird auf die Veröffentlichung von W.C. Griffin, J. Soc. Cosmetic Chem., Band 5, 249 (1954) hingewiesen.

Der Emulgatortyp und die Art und Weise der Zugabe des Emulgators beeinflussen die Polymerisation: es können dabei Unterschiede hinsichtlich Teilchengröße, Teilchengrößenverteilung, Stabilität der Copolymerisatdispersion sowie des Ausmaßes der Pfropfungsreaktionen beobachtet worden, beispielsweise in Abhängigkeit davon, ob der Emulgator vorgelegt wird oder ob er während der Copolymerisation zudosiert wird. Bevorzugte anionische Emulgatoren sind bei der Herstellung anionischer Emulsionscopolymerisate beispielsweise grenzflächenaktive Alkylsulfate, Alkylsulfonate, Alkylarylsulfate, Alkylarylsulfonate, Alkali- und/oder Ammoniumsalze von Alkyl- beziehungsweise Alkylarylmono- oder -polyglykolethersulfaten. Bevorzugte nichtionogene Emulgatoren sind beispielsweise oxethylierte Fettalkohole oder oxethylierte Alkylphenole. Besonders bevorzugt wird erfindungsgemäß Natriumlaurylsulfat, auch in Kombination mit Polysorbat 80, verwendet.

Die Menge an Tensiden bezogen auf das Polymerisat beträgt 0,05 bis 10 Gew.-%, bevorzugt 0,1 bis 5 Gew.-%.

Im Falle der Emulsionspolymerisation kann es von entscheidender Bedeutung sein, ob das Monomere für sich allein oder als wäßrige Emulsion zudosiert wird. Die wäßrige Emulsion der Monomeren enthält in aller Regel Wasser, anionische und/oder nichtionische Emulgatoren und/oder Schutzkolloide wie Polyvinylalkohol und ggf. weitere Schutzkolloide sowie ggf. Regler. Das Monomere bzw. eine Monomerenmischung oder die Monomer(en)emulsion werden zusammen mit dem Initiator, der i.a. in Lösung vorliegt, in einem Rührreaktor bei der Polymerisationstemperatur vorgelegt (batch-Prozeß), oder gegebenenfalls kontinuierlich oder in mehreren aufeinanderfolgenden Stufen in den Polymerisationsreaktor zudosiert (Zulaufverfahren). Beim Zulaufverfahren ist es üblich, daß der Reaktor vor Beginn der eigentlichen Polymerisation neben Wasser (um eine Rührung des Reaktors zu ermöglichen) bereits mit Teilmengen, selten der gesamten für die Polymerisation vorgesehenen Menge, der Einsatzstoffe wie Emulgatoren, Schutzkolloiden, Monomeren, Regler usw. oder Teilmengen der Zuläufe (i.a. Monomer- oder Emulsionszulauf sowie Initiatorzulauf) befüllt wird.

Zu berücksichtigen ist ferner, daß bei den jeweils verwendeten Comonomeren eine Copolymerisation untereinander prinzipiell möglich sein muß und daß sie auch tatsächlich erfolgt. Im einfachsten Fall läßt sich dies unter Zuhilfenahme der Copolymerisationsparameter beziehungsweise der Q- und e-Werte abschätzen (vgl. beispielsweise B. Brandrup, Immergut, Polymer Handbook, 2^{nd} od. (1975), John Wiley & Sons, New York).

So kann gegebenenfalls eine Copolymerisation unter Umständen dadurch machbar werden, daß man eine oder mehrere Monomerkomponenten vorlegt und das übrige Monomere beziehungsweise die übrige Monomermischung erst im Verlauf der Polymerisation zudosiert.

Der Feststoffgehalt der erhaltenen wäßrigen Polymerisat-Dispersionen bzw. Lösungen beträgt in der Regel 10 bis 70 Gew.-%, bevorzugt 20 bis 60 Gew.-%, besonders bevorzugt 25 bis 40 Gew.-%.

Die Polymerisat-Dispersionen oder Lösungen können durch verschiedenen Trocknungsverfahren wie z.B. Sprühtrocknung, Fluidized Spray Drying, Walzentrocknung oder Gefriertrocknung in Pulverform überführt werden. Durch die vorteilhaft geringe Viskosität der Polymerlösungen bzw. Dispersionen wird als Trocknungsverfahren bevorzugt die Sprühtrocknung eingesetzt. Aus dem so erhaltenen Polymer-Trockenpulver läßt sich durch Redispergieren in Wasser erneut eine wäßrige Dispersion bzw. Lösung herstellen. Die Überführung in Pulverform hat den Vorteil einer besseren Lagerfähigkeit, einer einfacheren Transportmöglichkeit sowie eine geringere Neigung für Keimbefall.

Die erfindungsgemäßen wasserlöslichen oder wasserdispergierbaren Copolymerisate eignen sich hervorragend als dispergierbare Filmbildner, Bindemittel, Benetzungshilfsstoff und/oder Löslichkeitsverbesserer für pharmazeutische Darreichungsformen.

Aufgrund der Flexibilität und der niedrigen Viskosität sind in der Regel keine zusätzlichen Weichmacher erforderlich.

Gegenstand der Erfindung sind daher auch pharmazeutische Darreichungsformen enthaltend mindestens ein erfindungsgemäßes wasserlösliches oder wasserdispergierbares Polymerisat als Überzugsmittel, Bindemittel und/oder filmbildender Hilfsstoff.

Bei den überzogenen Darreichungsformen handelt es sich bevorzugt u.a. um Filmtabletten, Filmmikrotabletten, Dragees, überzogene Pastillen, Kapseln, Kristalle, Granulate oder Pellets.

Bei den bindemittelhaltigen Darreichungsformen handelt es sich bevorzugt u.a. um Tabletten, Mikrotabletten, Kerne, Granulate oder Pellets.

Weiterhin können die erfindungsgemäßen Polymere zur Herstellung von Lösungen und Sprays verwendet werden, die, auf Haut oder Schleimhaut aufgebracht, einen Film ausbilden. Bedingt durch die enorme Dehnbarkeit und Adhäsivität haften die Filme lange auf der Haut oder Schleimhaut. Die Applikationsfrequenz kann so reduziert werden und der Tragekomfort ist erhöht. Beispiele hierfür sind Sprühverbände für Wunden, Desinfektionssprays, Lösungen mit Mycostatica, Mundsprays oder -lösungen mit Antibiotika etc. Aufgrund der Flexibilität ist auch der Einsatz bei transdermalen therapeutischen Systemen vorteilhaft.

Die erfindungsgemäß verwendeten Copolymerisate benetzen leicht lipophile Oberflächen und besitzen hervorragende Schutzkolloideigenschaften. Eingearbeitet in Suspensionen und Emulsionen lagern sie sich an die Teilchen der dispersen Phase und stabilisieren diese. Sie können daher als Benetzungshilfsmittel und Stabilisatoren in dispersen Systemen verwendet werden.

Durch Wechselwirkung mit schwer wasserlöslichen Arzneistoffen verbessern sie deren Löslichkeit und Lösungsgeschwindigkeit, wodurch Resorbierbarkeit und Bioverfügbarkeit der Arzneistoffe verbessert werden. Diese vorteilhafte Wirkung zeigt sich beispielsweise bei den Darreichungsformen, bei denen der Wirkstoff nicht gelöst vorliegt, wie z.B. Tabletten, Granulate, Suspensionen etc.

Die erfindungsgemäß verwendeten Polymere können gegebenenfalls auch in Kombination mit anderen Hilfsstoffen zusammen mit Wirkstoffen zu Polymer-Wirkstoffschmelzen verarbeitet werden, die entweder zu Arzneistoffen extrudiert und kalandriert werden oder nach der Extrusion zu Granulaten oder Pulvern zerteilt werden und erst anschließend in Arzneiformen verarbeitet werden, beispielsweise zu Tabletten verpreßt werden. Dabei bringen die Copolymerisate die bereits oben aufgeführten Eigenschaften in die Darreichungsform ein.

In verschiedenen pharmazeutischen Darreichungsformen können die erfindungsgemäßen Polymere folgende Funktionen hervorragend erfüllen:

Dispergierhilfsstoff, Suspendierhilfsstoff, Benetzungsmittel, Solubilisator für schwerlösliche Arzneistoffe, Emulgator, Kristallisationsinhibitor, Anticakinghilfsstoff, Schutzkolloid, Spreithilfsmittel, Viskositätsregulator, Hilfsstoff zur Herstellung von festen Lösungen mit Arzneistoffen, Hilfsstoff zur Einstellung der Wirkstofffreisetzung.

Bei der Verwendung zur Herstellung von Suppositorien und Vaginalglobuli gewährleisten die Polymere einerseits die Flexibilität der Darreichungsform und fördern andererseits den Zerfall und die Wirkstoffauflösung und sie kleiden die Schleimhaut mit einem wirkstoffhaltigen Film aus, der die Resorption verstärkt. Wie der Vergleich der Viskositäten der erfindungsgemäßen Polymere (Bsp. 1, Viskosität 77 mPas) mit entsprechenden Lösungen von Hydroxypropylmethylcellulose (Pharmacoat 606) (Bsp. 1, Viskosität 2000 mPas) zeigt, weisen die erfindungsgemäßen Polymere eine wesentlich niedrigere Viskosität auf.

Durch die niedrigen Viskositäten der Polymerisatlösungen können beim Coaten von Tabletten ebenso wie bei Bindemittelanwendungen konzentriertere Polymerzubereitungen eingesetzt werden, wodurch sich die Verfahren wesentlich kostengünstiger und zeitsparender gestalten lassen.

Die Auflösung bzw. Redispergierung der pulver- oder granulatförmigen Polymerisate zu wäßrigen Dispersionen bzw. Lösungen erfolgt wesentlich schneller als bei anderen Filmbildnern oder Bindemitteln, da die erfindungsgemäßen Polymerisate gut von Wasser benetzt werden, wenig klumpen und eine sehr hohe Auflösungsgeschwindigkeit aufweisen.

Magensaftlösliche Tabletten, die mit den Polymerisaten gecoatet wurden, zeigen eine gegenüber dem Kern nur geringfügig verlängerte Zerfallszeit, d.h. der Filmüberzug löst sich sehr schnell in künstlichem Magensaft auf.

Ferner wird durch die erfindungsgemäße Verwendung der Polymerisate die mechanische Festigkeit der Tabletten im Vergleich zu Hydroxypropylmethylcellulose sehr viel stärker erhöht.

Tabletten quellen in Abhängigkeit von den verwendeten Hilfsund Wirkstoffen, der Lagerzeit und den Lagerbedingungen, wie Temperatur und Feuchtigkeit, unterschiedlich stark. Ein starrer Filmüberzug erleidet bei Quellung des Kernes Risse. Deshalb ist die Elastizität von Filmbildnern eine wichtige Größe. Die erfindungsgemäßen Copolymerisate besitzen eine ausgesprochen hohe Flexibilität und Elastizität. So kann die Reißdehnung bis zu 300 % betragen. Eine Rißbildung ist daher auch bei starker Kernquellung nicht zu erwarten.

Die Polymere können in reiner Form oder aber zusammen mit den üblichen Hilfsstoffen auf den wirkstoffhaltigen Kern appliziert werden. Übliche Hilfsstoffe sind z.B. Farbpigmente zur Einfärbung, Weißpigmente, wie Titandioxid, zur Erhöhung der Deckkraft, Talkum und Siliciumdioxid als Antiklebemittel, Polyethylenglykole, Glycerin, Propylenglykol, Triacetin, Triethylcitrat als Weichmacher und verschiedene oberflächenaktive Stoffe, wie Natriumlaurylsulfat, Polysorbat 80, Pluronics und Cremophore, zur Verbesserung des Benetzungsverhaltens. Die exemplarisch genannten Stoffe stellen keine Begrenzung dar. Es können alle bekanntermaßen für magensaftlösliche Filmüberzüge geeigneten Zusatzstoffe verwendet werden.

Es ist ferner möglich, die erfindungsgemäß verwendeten Polymerisate mit anderen Filmbildnern bzw. Polymeren im Verhältnis 1:9 bis 9:1 zu kombinieren.

Hierzu können beispielsweise folgende Polymere eingesetzt werden:

Polyvinylpyrrolidon, Polyvinylpyrrolidon-Copolymere, wasserlösliche Cellulosederivate, wie Hydroxypropylcellulose, Hydroxypropylmethylcellulose, Methylcellulose, Hydroxyethylcellulose, Acrylat- und Methacrylat-Copolymere, Polyvinylalkohole, Polyethylenglykole, Polyethylenoxid-Polypropylenoxid-Blockpolymere.

Als Überzugsverfahren lassen sich die gebräuchlichen Verfahren, wie das Coaten in der Wirbelschicht oder im Horizontaltrommelcoater, das Tauchschwertverfahren und das Kesselcoatingverfahren, anwenden. Neben der Anwendung auf Tabletten können die erfindungsgemäßen Polymere auch für das Coating von anderen pharmazeutischen Zubereitungen, wie Granulaten, Pellets, Kristallen oder Kapseln, eingesetzt werden. Die neuen Überzugsmittel werden wie üblich in einer Stärke von 5 bis 200 µm, vorzugsweise 10 bis 100 µm, aufgetragen.

Bei der Verwendung als Bindemittel unterscheidet man je nach Verarbeitungsverfahren zwischen Feucht- und Trockenbindemittel. Letztere werden u.a. bei der Direkttablettierung und bei der Trockengranulation bzw. Kompaktierung verwendet. Hierbei wird das Bindemittel mit dem Wirkstoff und gegebenenfalls weiteren Hilfsstoffen vermischt und anschließend direkttablettiert oder granuliert bzw. kompaktiert.

Im Gegensatz dazu wird bei der Feuchtgranulation die Wirkstoff-Hilfsstoffmischung mit einer Lösung des Bindemittels in Wasser oder einem organischen Lösungsmittel befeuchtet, die feuchte Masse durch ein Sieb gegeben und anschließend getrocknet. Befeuchtung und Trocknung können dabei auch parallel ablaufen, wie z.B. in der Wirbelschichtgranulation.

Für eine optimale Verarbeitung soll das Bindemittel in Lösung niedrigviskos sein, da viskose Lösungen zu inhomogenen Granulaten führen.

Ein Bindemittel soll zu gleichmäßigen, harten, abriebstabilen Granulaten bzw. Tabletten führen. Insbesondere bei Tabletten kommt der Bruchfestigkeit besondere Bedeutung zu, da sich viele Wirkstoffe schlecht verpressen lassen und somit Tabletten mit unzureichender mechanischer Stabilität ergeben.

Weiterhin soll der Zerfall der Arzneiformen sowie die Freisetzungsgeschwindigkeit der Wirkstoffe durch das Bindemittel nicht nennenswert nachteilig beeinflußt werden.

Die gängigsten Bindemittel sind beispielsweise Polyvinylpyrrolidon, Vinylacetat-Vinylpyrrolidon Copolymere, Gelatine, Stärkekleister, Maltodextrine, hydroxyalkylierte bzw. carboxyalkylierte Cellulosederivate, wie Hydroxypropylmethylcellulose, Methylcellulose, Natriumcarboxymethylcellulose sowie natürliche Gummisorten, wie beispielsweise Gummi Arabicum, Pektin oder Alginat.

Viele dieser Bindemittel weisen in Lösung eine hohe Viskosität auf und sind schlecht verarbeitbar. Durch die hohe Viskosität werden die zu granulierenden Pulverteilchen schlecht und ungleichmäßig benetzt, so daß daraus eine zu geringe Granulatfestigkeit und eine ungünstige Korngrößenverteilung resultieren.

Viele Bindemittel sind zudem hygroskopisch und quellen bei Wasseraufnahme. Dadurch können sich die Granulat- und Tabletteneigenschaften dramatisch verändern.

Überraschenderweise wurde nun gefunden, daß die erfindungsgemäßen Polymerisate über ausgezeichnete Bindemittelwirkungen verfügen und zudem in Konzentrationsbereichen von 0,5 bis 20 Gew.-%, bevorzugt 1 bis 10 Gew.-% der Gesamtmenge der Formulierung den Zerfall nicht nennenswert beeinflussen. Aufgrund des guten Benetzungsverhaltens der Copolymerisate kann sich zudem die Freisetzung von schlecht löslichen Wirkstoffen verbessern.

Mit den Copolymeren als Bindemittel ergeben sich mechanisch außerordentlich stabile und auch über lange Lagerzeiten stabile Granulate bzw. Tabletten.

In den nachfolgenden Beispielen wird die Herstellung und Verwendung der erfindungsgemäßen Copolymerisate näher erläutert, ohne die Erfindung jedoch auf die Ausführungsbeispiele einzuschränken.

### Beispiel 1

| | | |
|---|---|---|
| Zusammensetzung | 35 Gew.-% | Mowiol 4-88 (Polyvinylalkohol, Fa. Clariant) |
| | 55 Gew.-% | Hydroxyethylmethacrylat |
| | 10 Gew.-% | Methylmethacrylat |
| | | |
| Vorlage | 230,0 g | VE-Wasser (vollentsalztes Wasser) |
| | 0,7 g | Natriumlaurylsulfat |
| | 101,5 g | Mowiol® 4-88 |
| | 30 ml | von Zulauf 1 |
| | | |
| Zulauf 1 | 550,0 g | VE-Wasser |
| | 0,3 g | Natriumlaurylsulfat |
| | 133,3 g | Mowiol® 4-88 als 30 %ige wäßrige Lösung |
| | 220,0 g | Hydroxyethylmethacrylat |
| | 40,0 g | Methylmethacrylat |
| | | |
| Zulauf 2 | 5,0 g | Natriumpersulfatlösung als 7 %ige wäßrige Lösung |
| | | |
| Zulauf 3 | 30,0 g | Natriumpersulfatlösung als 7 %ige wäßrige Lösung |
| | 55,0 g | VE-Wasser |

Apparatur: 2-1-Pilotrührwerk, Ölbad, Ankerrührer, Prozeßleitsystem für Zuläufe

Die Apparatur ist mit Stickstoff gespült

### Fahrweise

Die Vorlage wurde auf 80°C Innentemperatur aufgeheizt. Bei ca. 75°C wurde Zulauf 2 zudosiert und 15 Minuten anpolymerisiert. Zulauf 1 wurde in 1,5 h, Zulauf 3 gleichzeitig in 10 Minuten zugegeben. Nach Ende des Zulaufs 1 wurde noch 3 h bei 80°C nachpolymerisiert. Anschließend wurde der Ansatz abgekühlt und über 120 µm filtriert.

| | |
|---|---|
| Feststoffgehalt | 28,9 Gew.-% |
| Mittlere Teilchengröße | 325 nm |
| Koagulat | 0,1 g |
| | |
| Viskosität (20 % ig) | 77 mPas |
| | |
| Filmeigenschaften.(54 % rel. F., 23°C) | |
| Reißdehnung | 43 % |
| Reißfestigkeit | 45 N/mm² |
| | |
| Vergleich Pharmacoat® 606 (Fa. Shin-etsu) | |
| Viskosität (20 %ig) | 2000 mPas |
| | |
| Filmeigenschaften (54 % rel. F.,23°C) | |
| Reißdehnung | 17 % |
| Reißfestigkeit | 58 N/mm² |

### Anwendungsbeispiel

### Herstellung von Propranolol-HCl Filmtabletten (magensaftlöslicher Überzug)

Auf 9 mm gewölbte Tablettenkerne mit 40 mg Propranolol-HCl (Fa. Knoll AG), 195,0 mg Ludipress® (Fa. BASF Aktiengesellschaft), 12,50 mg Kollidon® VA 64 (Fa. BASF Aktiengesellschaft) und 2,50 mg Magnesiumstearat wurde in einem Horizontaltrommelcoater (Accela-Cota 24", Fa. Manesty) ein Filmüberzug gemäß folgender Zusammensetzung aufgesprüht:

| | |
|---|---|
| Polymer aus Beispiel 1 Polyvinylalkohol/Hydroxyethylmethacrylat/Methylmethacrylat | 12,0 Gew.-% |
| Sicovit® rot (Fa. BASF Aktiengesellschaft) | 1,5 Gew.-% |
| Titandioxid BN 56 (Fa. Kronos) | 3,0 Gew.-% |
| Talkum Pulver (Fa. Riedel de Haen) | 4,5 Gew.-% |
| Wasser | 79,0 Gew.-% |

Zur Herstellung der Sprühdispersion wurde das sprühgetrocknete Polymerisat aus Beispiel 1 in Wasser unter Rühren redispergiert, mit Sicovit® rot, Titandioxid und Talkum versetzt und anschließend in einer Korundscheibenmühle homogenisiert. 1090 g (incl. eines Zuschlages von 10 % für Sprühverluste) wurden bei einer Zulufttemperatur von 55°C und einer Sprührate von 31 g/min mit einer 1,0 mm weiten Sprühdüse und einem Sprühdruck von 1,8 bar auf 5 000 g Kerne appliziert. Nach der Sprühung wurde noch 5 min. bei 55°C nachgetrocknet.

Man erhielt glatte, glänzende, rote Filmtabletten mit folgenden Eigenschaften:

| | |
|---|---|
| Aussehen | sehr glatte Oberfläche, schön ausgebildete Gravur |
| Zerfall (künstl. Magensaft) | 5 min. 13 s. |
| Zerfallszeitdifferenz (Filmtablette-Kern) | 55 s. |
| Bruchfestigkeit | 94 N |
| Bruchfestigkeitsdifferenz (Filmtablette-Kern) | 24 N |

## Patentansprüche

1. Wasserlösliche oder wasserdispergierbare Copolymere erhältlich durch radikalisch initiierte Polymerisation, von
a) 80 bis 20 Gew.-% Hydroxy-C₁-C₆-alkyl(meth)acrylat sowie gegebenenfalls einer oder mehreren Verbindungen der Formel (A) oder (B) 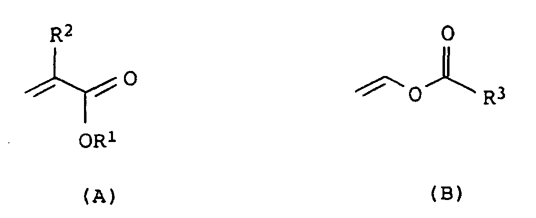 mit R¹ = H, C₁-C₆-Alkyl,
R² = H, CH₃
R³ = C₁-C₂₄-Alkyl
oder Gemischen davon
in Gegenwart von
b) 20 bis 80 Gew.-% Polyvinylalkohol (PVA) und
c) gegebenenfalls 0 bis 20 Gew.-% weiterer polymerisierbarer Verbindungen (C).

2. Wasserlösliche oder wasserdispergierbare Copolymere gemäß Anspruch 1, **dadurch gekennzeichnet, dass** die radikalisch initiierte Polymerisation eine Emulsionspolymerisation ist.

3. Wasserlösliche oder wasserdispergierbare Copolymere gemäß einem der Ansprüche 1 oder 2, **dadurch gekennzeichnet, daß** als Hydroxy-C₁-C₆-alkyl(meth)acrylat Hydroxyethylmethacrylat eingesetzt wird.

4. Wasserlösliche oder wasserdispergierbare Copolymere gemäß einem der Ansprüche 1 bis 3, **dadurch gekennzeichnet, daß** die Verbindungen der Formel (A), ausgewählt sind aus der Gruppe Methylmethacrylat, Ethylacrylat, Methylacrylat, oder Gemischen davon.

5. Wasserlösliche oder wasserdispergierbare Copolymere gemäß einem der Ansprüche 1 bis 4, **dadurch gekennzeichnet, daß** die Verbindungen der Formel (B), ausgewählt sind aus der Gruppe der C₃-C₂₄-Vinylester.

6. Verfahren zur Herstellung von wasserlöslichen oder wasserdispergierbaren Copolymeren gemäß einem der Ansprüche 1 bis 5, durch radikalisch initiierte Polymerisation in einem wäßrigen oder nichtwäßrigen aber wassermischbaren Lösungsmittel oder in gemischt nichtwäßrigen/wäßrigen Lösungsmitteln.

7. Verfahren gemäß Anspruch 6, **dadurch gekennzeichnet, daß** die Polymerisation in Gegenwart von 30 bis 55 Gew.-% Polyvinylalkohol stattfindet.

8. Pharmazeutische Darreichungsformen enthaltend mindestens ein wasserlösliches oder wasserdispergierbares Copolymer gemäß einem der Ansprüche 1 bis 5, als Überzugs-, Bindemittel und/oder filmbildender Hilfsstoff.

9. Verwendung von wasserlöslichen oder wasserdispergierbaren Copolymeren gemäß einem der Ansprüche 1 bis 5, als Überzugs-, Bindemittel und/oder filmbildender Hilfsstoff in pharmazeutischen Darreichungsformen.

## Claims

1. A water-soluble or water-dispersible copolymer obtainable by free-radical polymerization of
a) 80 to 20% by weight of hydroxy-C₁-C₆-alkyl (meth)acrylate and, where appropriate, one or more compounds of the formula (A) or (B) 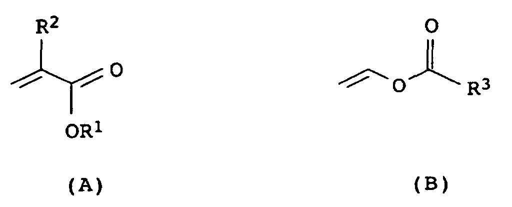 with R¹ = H, C₁-C₆-alkyl,
R² = H, CH₃
R³ = C₁-C₂₄-alkyl
or mixtures thereof
in the presence of
b) 20 to 80% by weight of polyvinyl alcohol (PVA) and
c) where appropriate 0 to 20% by weight of other polymerizable compounds (C).

2. A water-soluble or water-dispersible copolymer as claimed in claim 1, wherein the free-radical polymerization is an emulsion polymerization.

3. A water-soluble or water-dispersible copolymer as claimed in either of claims 1 or 2, wherein hydroxyethyl methacrylate is employed as hydroxy-C₁-C₆-alkyl (meth)acrylate.

4. A water-soluble or water-dispersible copolymer as claimed in any of claims 1 to 3, wherein the compounds of the formula (A) are selected from the group of methyl methacrylate, methyl acrylate, methyl acrylate, or mixtures thereof.

5. A water-soluble or water-dispersible copolymer as claimed in any of claims 1 to 4, wherein the compounds of the formula (B) are selected from the group of C₃-C₂₄ vinyl esters.

6. A process for preparing water-soluble or water-dispersible copolymers as claimed in any of claims 1 to 5 by free-radical polymerization in an aqueous or nonaqueous but water-miscible solvent or in mixed nonaqueous/aqueous solvents.

7. A process as claimed in claim 6, wherein the polymerization takes place in the presence of from 30 to 55% by weight of polyvinyl alcohol.

8. A pharmaceutical dosage form comprising at least one water-soluble or water-dispersible copolymer as claimed in any of claims 1 to 5 as coating agent, binder and/or film-forming excipient.

9. The use of water-soluble or water-dispersible copolymers as claimed in any of claims 1 to 5 as coating agent, binder and/or film-forming excipient in pharmaceutical dosage forms.

## Revendications

1. Copolymères solubles ou dispersables à l'eau obtenus par polymérisation radicalaire de :
a) 80 à 20% en poids d'un (méth)acrylate d'hydroxyalkyle en C₁-C₆ et le cas échéant d'un ou plusieurs composés de formule (A) ou (B) 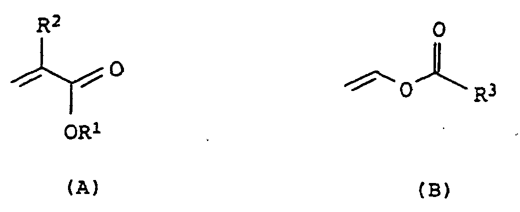 dans laquelle
R¹ = H, alkyle en C₁-C₆,
R² = H, CH₃,
R³ = alkyle en C₁-C₂₄
ou leurs mélanges en présence de
b) 20 à 80% en poids d'alcool polyvinylique et
c) le cas échéant 0 à 20% en poids d'autres composés polymérisables (C).

2. Copolymères solubles ou dispersables à l'eau selon la revendication 1, **caractérisés par le fait que** la polymérisation radicalaire est une polymérisation en émulsion.

3. Copolymères solubles ou dispersables à l'eau selon l'une des revendications 1 ou 2, **caractérisés par le fait que** le (méth)acrylate d'hydroxyalkyle en C₁-C₆ est le méthacrylate d'hydroxyéthyle.

4. Copolymères solubles ou dispersables à l'eau selon l'une des revendications 1 à 3, **caractérisés par le fait que** les composés de formule (A) sont choisis dans le groupe consistant en le méthacrylate de méthyle, l'acrylate d'éthyle, l'acrylate de méthyle ou leurs mélanges.

5. Copolymères solubles ou dispersables à l'eau selon l'une des revendications 1 à 4, **caractérisés par le fait que** les composés de formule (B) sont choisis dans le groupe des esters vinyliques en C₃-C₂₄·

6. Procédé pour la préparation des copolymères solubles ou dispersables à l'eau selon l'une des revendications 1 à 5 par polymérisation radicalaire dans un solvant aqueux ou non aqueux mais miscible à l'eau ou dans un mélange de solvants non aqueux/aqueux.

7. Procédé selon la revendication 6, **caractérisé par le fait que** la polymérisation est réalisée en présence de 30 à 55% en poids d'alcool polyvinylique.

8. Formes d'administration pharmaceutiques contenant au moins un copolymère soluble ou dispersable à l'eau selon l'une des revendications 1 à 5 en tant que produit de revêtement, liant et/ou produit auxiliaire filmogène.

9. Utilisation des copolymères solubles ou dispersables à l'eau selon l'une des revendications 1 à 5 en tant que produits de revêtement, liants et/ou produits auxiliaires filmogènes dans des formes d'administration pharmaceutiques.
